# EUROPEAN PATENT SPECIFICATION

(11) **EP 3 424 282 B1**
(45) Date of publication and mention of the grant of the patent: **06.04.2022**
(21) Application number: 17713780.9
(22) Date of filing: 28.02.2017
(51) Int. Cl.: H05K 1/03, H01L 21/283, H01L 51/00, H05K 1/16, H01L 23/14, H01L 23/498, H05K 1/18, H05K 3/46

(54) **STICKER ELECTRONICS**
AUFKLEBERELEKTRONIK
AUTOCOLLANTS ÉLECTRONIQUES

(30) Priority: 29.02.2016 US 201662301611 P
(43) Date of publication of application: 09.01.2019
(73) Proprietor: King Abdullah University of Science and Technology, Thuwal 23955-6900 (SA)
(72) Inventor: HUSSAIN, Muhammad Mustafa, Thuwal 23955-6900 (SA); TORRES SEVILLA, Galo Andres, Thuwal 23955-6900 (SA); DIAZ CORDERO, Marlon Steven, Thuwal, 23955-6900 (SA)
(74) Representative: Ipsilon
(86) International application number: PCT/IB2017/051170
(87) International publication number: WO 2017/149455

(56) References cited:
- EP-A1- 2 966 945
- WO-A1-2009/096802
- GB-A- 2 494 223
- ADAM C SIEGEL ET AL: "Foldable Printed Circuit Boards on Paper Substrates", ADVANCED FUNCTIONAL MATERIALS, WILEY - V C H VERLAG GMBH & CO. KGAA, DE, vol. 20, no. 1, 8 January 2010 (2010-01-08), pages 28-35, XP001551577, ISSN: 1616-301X, DOI: 10.1002/ADFM.200901363 [retrieved on 2009-10-15]
- PUSHPARAJ V L ET AL: "Flexible energy storage devices based on nanocomposite paper", PROCEEDINGS NATIONAL ACADEMY OF SCIENCES, NATIONAL ACADEMY OF SCIENCES, US, vol. 104, no. 34, 21 August 2007 (2007-08-21), pages 13574-13577, XP008110890, ISSN: 0027-8424, DOI: 10.1073/PNAS.0706508104

## Description

### CROSS-REFERENCE TO RELATED PATENT APPLICATIONS

### FIELD OF THE DISCLOSURE

The instant disclosure relates to electronic devices. More specifically, portions of this disclosure relate to small electronic devices packaged as a sticker.

### BACKGROUND

Electronic devices are continually shrinking in size and providing new capabilities with smaller footprints. For example, televisions (TVs) have evolved from tube-based black-and-white devices of several feet in thickness to full-color high-definition displays that are only fractions of an inch in thickness. In any device or apparatus that does not have storage space as part of its functionality, there is generally little reason to not further reduce the size of that device or apparatus. Conventional semiconductor manufacturing has worked to reduce the size of many devices. However, conventional semiconductor manufacturing technology is not well suited to all kinds of devices, in part because of the need for a rigid substrate, such as a silicon wafer.

### SUMMARY

Flexible substrates can be much easier for constructing consumer device and provide more desirable characteristics and reduced cost. Some techniques for manufacturing flexible semiconductor substrates are described in U.S. Patent No. 9,209,083 to Hussain et al. and entitled "Integrated Circuit Manufacturing for Low-Profile and Flexible Devices" and U.S. Patent Application Publication No. 2014/0239459 to Hussain et al. and entitled "Method for Producing Mechanically Flexible Silicon Substrate," both of which are incorporated by reference in their entirety. These techniques are example techniques that may be employed in manufacturing the devices, such as the electronic stickers, described below. Other techniques may also be suitable for manufacturing these devices.

Layers of devices may be stacked on each other on a flexible substrate to form a flexible device, such as an electronic sticker. Different functionality can be incorporated onto the different layers and packaged together as a low-cost electronic device, which may be used as in an Internet of Things (IoT) network. Some example functionality for the electronic stickers includes lighting, control, power charging, and environmental sensing. Further, functionality can be combined, such as to provide for controllable lighting in a single electronic sticker.

The invention is defined in the apparatus claim 1 and in the method claim 4.

In certain embodiments, at least one layer of the one or more layers may include an inductive power coil configured to receive power for operating the one or more electronic devices; at least one layer of the one or more layers may include one or more light emitting diodes (LEDs); the light emitting diodes (LEDs) may be configured to receive power from the inductive power coil; at least one layer of the one or more layers may include a wireless module; the wireless module may be coupled to the inductive power coil and to the light emitting diodes (LEDs); the wireless module may be configured to receive commands to operate the light emitting didoes (LEDs) and to operate the light emitting diodes (LEDs) based, at least in part, on the received commands; the apparatus may include an adhesive backing on the flexible substrate to form a sticker; the apparatus may also include a flexible battery coupled to the one or more layers; the flexible substrate may be packaged using polymer on cellulose manufacturing using 3D printing; at least one layer of the one or more layers may include one or more sensors; the one or more sensors may be configured to receive power from the inductive power coil; at least one layer of the one or more layers may include a wireless module; the wireless module may be coupled to the inductive power coil and to the one or more sensors; the wireless module may be configured to receive data from the one or more sensors and to wirelessly communicate the received data to a client device; the one or more sensors may include at least one of a smoke detector and a carbon monoxide detector; and/or the one or more sensors may include a rain gauge.

The foregoing has outlined rather broadly certain features and technical advantages of embodiments of the present invention in order that the detailed description that follows may be better understood. Additional features and advantages will be described hereinafter that form the subject of the claims of the invention. It should be appreciated by those having ordinary skill in the art that the conception and specific embodiment disclosed may be readily utilized as a basis for modifying or designing other structures for carrying out the same or similar purposes. It should also be realized by those having ordinary skill in the art that such equivalent constructions do not depart from the spirit and scope of the invention as set forth in the appended claims. Additional features will be better understood from the following description when considered in connection with the accompanying figures. It is to be expressly understood, however, that each of the figures is provided for the purpose of illustration and description only and is not intended to limit the present invention.

### BRIEF DESCRIPTION OF THE DRAWINGS

For a more complete understanding of the disclosed system and methods, reference is now made to the following descriptions taken in conjunction with the accompanying drawings.
FIGURE 1 is a top view of an electronic sticker with light emitting diodes (LEDs) according to one embodiment of the disclosure.
FIGURE 2 is side view of the electronic sticker with LEDs of FIGURE 1.
FIGURE 3 is a partially exploded perspective view of the electronic sticker with LEDs of FIGURE 1.
FIGURE 4 is a flowchart of a method of making an electronic sticker according to one embodiment of the disclosure.

### DETAILED DESCRIPTION

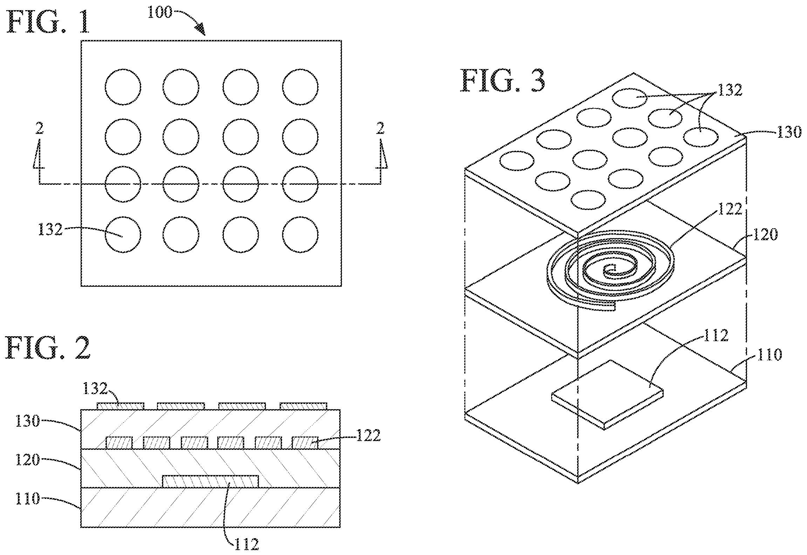

Electronic stickers may be manufactured on flexible substrates as layers and packaged together. The package may then have an adhesive applied to one side to provide capability for sticking the electronic devices to surfaces. The stickers can be wrappable, placed on surfaces, glued on walls or mirrors or wood or stone, and have electronics which may or may not be ultrathin. Packaging for the electronic sticker can use polymer on cellulose manufacturing and/or three dimensional (3-D) printing. In one example, the electronic stickers are used to provide lighting capability as shown in FIGURES 1-3. FIGURE 1 is a top view of an electronic sticker 100 with light emitting diodes (LEDs) according to one embodiment of the disclosure. FIGURE 2 is side view of the electronic sticker 100 with LEDs of FIGURE 1. FIGURE 3 is a partially exploded perspective view of the electronic sticker 100 with LEDs of FIGURE 1. An electronic sticker 100 may include layers 110, 120, and 130. Although three layers are shown in FIGURE 1, an electronic sticker may include more or less layers.

The first layer 110 may include a wireless module 112, such as an integrated circuit (IC) including a controller or processor and associated radio circuitry for performing wireless communications. The wireless module 112 may provide support for wireless communications according to IEEE 802.11 WiFi, Bluetooth, Long Term Evolution (LTE), and/or other wireless communication standards.

The second layer 120 may include a power coil 122 for receiving energy to power electronic components on the layers 110, 120, and/or 130. For example, the power coil 122 may be inductively coupled to a permanent or mobile power source to energize the system 100. In one example, the system 100 may be applied as a sticker to a receiving element that is affixed to an infrastructure power system, such as the electrical system in a home. In another example, the system 100 may be applied as a sticker at a location and a mobile power unit or a mobile phone brought into proximity with the system 100 to temporarily power the system 100. In one embodiment, a flexible battery (not shown) may be integrated with the system 100 or an external battery (not shown) may be attached to the system 100. The battery may be coupled to the power coil 122 to receive energy to be stored. The system 100 can then be operated in the absence of a power source for short or long durations of time based on a capacity of the battery. Although a battery is described, other configurations may be integrated with and attached to the system 100 for energy storage. For example, metal-insulator-metal (MIM) capacitors (not shown) may be manufactured in a layer of the system 100 and used to store energy received from the coil 122.

The third layer 130 may include light emitting diodes (LEDs) 132 that convert energy to visible light to illuminate areas. The LEDs 132 may be coupled to other layers, such as to the power coil 122 on the second layer 120 and to the wireless module 112 on the first layer 110. The LEDs 132 may receive energy from the power coil 122 under control of the wireless module 112. For example, a switch (not shown) may couple the LEDs 132 to the power coil 122, and the switch may be toggled on and off by the wireless module 112 to turn on and off the LEDs 132. As another example, dimmer circuitry (not shown) may be coupled between the LEDs 132 and the power coil 122 and controlled by the wireless module 112 to control an intensity of the light output of the LEDs 132.

The wireless module 112 may be controlled from a local device, such as a user's mobile phone located in the room with the LEDs 132. In one scenario, a user may tap their mobile phone to the electronic sticker, upon which the power coil 122 is energized by the battery in the mobile phone to activate the wireless module 112. The user's mobile phone then pairs with the wireless module 112, upon which a control application activates on the user's mobile phone. The control application may include a power button for turning on and off the LEDs 132, a dimmer slider for controlling an intensity of the LEDs 132, and/or a color selector for selecting a color of light emitted by the LEDs 132. The wireless module 112 may also or alternatively be controlled from a remote device, such as a user's laptop while the user is away from their home.

Although a light emitting electronic sticker is described above, other functionality may be provided in the various layers of the system 100. In another embodiment, an electronic sticker may be configured as a smoke detector. Smoke sensors may be integrated in a third layer 130 that is outward facing towards the environment around the sticker. The smoke sensors may be coupled to a wireless module that can communicate information from the smoke sensors, such as the presence of smoke in the area. Further, although smoke sensors are described, any sensor may be integrated in the electronic sticker. For example, a carbon monoxide detector may be included with or in the alternate to the smoke sensor. As another example, a water sensor may be integrated with an electronic sticker and used to detect leaky pipes or overflowing tubs and sinks. In yet another example, a temperature sensor or ultraviolet

(UV) light sensor may be integrated in an electronic sticker. The sticker may be placed in rooms throughout a house, on windows throughout the house, and/or on a patio or in the yard. A user may activate the sensors with their mobile phone to obtain measurements from the sensors integrated in the stickers. For example, a user may tap their phone against the electronic sticker to energize the power coil and pair with the wireless module, which then transmits to the user's phone a measurement from the integrated sensor, such as the local temperature. The user's mobile device may then store the measurement for later viewing or automatically launch an application that provides the information to the user. In another example, a rain gauge sensor may be integrated in an electronic sticker. The rain gauge may be used to determine recent rainfall amounts and the wireless module activated to send commands to a sprinkler system.

In yet another embodiment, the electronic stickers may provide services to other devices. For example, an electronic sticker may provide recharging capability to mobile devices. Such an electronic sticker may include the power coil 122 shown in FIGURE 1, but use the power coil 122 to transmit energy to other devices. The electronic sticker may be hard-wired to an electrical system, such as a house's 120V AC electrical system. One layer of the electronic sticker may include appropriate circuitry to generate an appropriate level signal for the power coil 122. When a user's mobile device is placed against the sticker, the user's mobile device may receive energy to recharge its battery. Although similar charging pads are conventionally available, they all involve rigid structures that are not suitable in all areas and that are bulky and unsightly. When manufactured as an electronic sticker, such a charging device may be ultrathin, conform to surface shapes, and adhere to the surface in a much smaller package than conventional wireless charging pads.

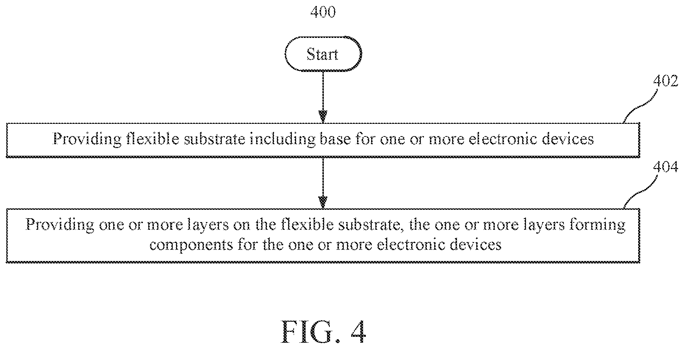

FIGURE 4 is a flowchart of a method 400 of making an electronic sticker according to one embodiment of the disclosure. In operation 402, a flexible substrate comprising a base for one or more electronic devices may be provided. For example, first layer 110 may form the flexible substrate for the one or more electronic devices. In operation 404, one or more layers may be provided on the flexible substrate. The one or more layers may form components for the one or more electronic devices. A top layer of the one or more layers may be exposed to an environment around the top layer and include components to interact with the environment. For example, third layer 130 may form the top layer of the one or more layers.

At least one layer of the one or more layers may include an inductive power coil configured to receive power for operating the one or more electronic devices. For example, second layer 120 may include power coil 122 for receiving energy to power electronic components on the layers 110, 120, and/or 130. At least one layer of the one or more layers may include one or more light emitting diodes (LEDs). The LEDs may be configured to receive power from the inductive power coil. For example, the third layer 130 may include LEDs 132 that may receive energy from the power coil 122. At least one layer of the one or more layers may include a wireless module. The wireless module may be coupled to the inductive power coil and to the LEDs. For example, the LEDs may receive energy from the power coil 122 under control of the wireless module 112. The wireless module may be configured to receive commands to operate the LEDs and to operate the light emitting diodes LEDs based, at least in part, on the received commands.

The method 400 may further include providing an adhesive backing on the flexible substrate to form a sticker. The method 400 may further include coupling a flexible battery to the one or more layers. The method 400 may further include packaging the flexible substrate using polymer on cellulose manufacturing using 3D printing.

At least one layer of the one or more layers may include one or more sensors. The one or more sensors may be configured to receive power from the inductive power coil. At least one layer of the one or more layers may include a wireless module coupled to the inductive power coil and to the one or more sensors. The wireless module may be configured to receive data from the one or more sensors and to wirelessly communicate the received data to a client device. The one or more sensors may include at least one of a smoke detector and a carbon monoxide detector. For example, third layer 130 may include a smoke sensor integrated therein. The one or more sensors may include a rain gauge.

## Claims

1. An apparatus (100), comprising:
a flexible substrate (110) comprising a base for one or more electronic devices (112, 122, 132); and
one or more layers (120, 130) on the flexible substrate (110), wherein the one or more layers form components for the one or more electronic devices (112, 122, 132),
wherein a top layer (130) of the one or more layers (120, 130) is exposed to an environment around the apparatus (100) and includes components to interact with the environment, wherein a second layer of the one or more layers comprises an inductive power coil configured to receive power for operating the one or more electronic devices, wherein the top layer of the one or more layers comprises one or more light emitting diodes, wherein the light emitting diodes are configured to receive power from the inductive power coil, wherein a first layer of the one or more layers comprises a wireless module, wherein the wireless module is coupled to the inductive power coil and to the light emitting diodes, wherein the wireless module is configured to receive commands to operate the light emitting didoes and to operate the light emitting diodes based, at least in part, on the received commands, and wherein, when a mobile phone is tapped on the apparatus, the power coil is configured to be energized by a battery of the mobile phone and to activate the wireless module, which is paired with the mobile phone to control the light emitting diodes.

2. The apparatus of claim 1, wherein the apparatus further comprises an adhesive backing on the flexible substrate to form a sticker.

3. The apparatus of claim 1, further comprising a flexible battery coupled to the one or more layers.

4. A method, comprising:
providing (402) a flexible substrate (110) comprising a base for one or more electronic devices (112, 122, 132); and
providing (404) one or more layers (120, 130) on the flexible substrate (110), wherein the one or more layers (120, 130) form components for the one or more electronic devices (112, 122, 132),
wherein a top layer (130) of the one or more layers (120, 130) is exposed to an environment around the top layer (130) and includes components to interact with the environment, wherein a second layer of the one or more layers comprises an inductive power coil configured to receive power for operating the one or more electronic devices, and wherein the top layer of the one or more layers comprises one or more light emitting diodes, wherein the light emitting diodes are configured to receive power from the inductive power coil, wherein a first layer of the one or more layers comprises a wireless module, wherein the wireless module is coupled to the inductive power coil and to the light emitting diodes, and wherein the wireless module is configured to receive commands to operate the light emitting didoes and to operate the light emitting diodes based, at least in part, on the received commands,
wherein, when a mobile phone is tapped on the apparatus, the power coil is configured to be energized by a battery of the mobile phone and to activate the wireless module, which is paired with the mobile phone to control the light emitting diodes.

5. The method of claim 4, further comprising providing an adhesive backing on the flexible substrate to form a sticker.

6. The method of claim 4, further comprising coupling a flexible battery to the one or more layers.

7. The method of claim 4, further comprising packaging the flexible substrate using polymer on cellulose manufacturing using 3D printing.

## Patentansprüche

1. Vorrichtung (100), umfassend:
ein flexibles Substrat (110), das eine Basis für ein oder mehrere elektronische Bauelemente (112, 122, 132) umfasst; und
eine oder mehrere Schichten (120, 130) auf dem flexiblen Substrat (110), wobei die eine oder die mehreren Schichten Komponenten für das eine oder die mehreren elektronischen Bauelemente (112, 122, 132) bilden,
wobei eine obere Schicht (130) der einen oder der mehreren Schichten (120, 130) zu einer Umgebung um die Vorrichtung (100) freiliegend ist und Komponenten zum Interagieren mit der Umgebung umfasst, wobei eine zweite Schicht der einen oder der mehreren Schichten eine Induktionsleistungsspule umfasst, die zum Empfangen von Leistung zum Betreiben des einen oder der mehreren elektronischen Bauelemente ausgelegt ist, wobei die obere Schicht der einen oder der mehreren Schichten eine oder mehrere Leuchtdioden umfasst, wobei die Leuchtdioden zum Empfangen von Leistung von der Induktionsleistungsspule ausgelegt sind, wobei eine erste Schicht der einen oder der mehreren Schichten ein Drahtlosmodul umfasst, wobei das Drahtlosmodul mit der Induktionsleistungsspule und mit den Leuchtdioden gekoppelt ist, wobei das Drahtlosmodul so ausgelegt ist, dass es Befehle zum Betreiben der Leuchtdioden empfängt und die Leuchtdioden wenigstens zum Teil basierend auf den empfangenen Befehlen betreibt, und wobei die Leistungsspule so konfiguriert ist, dass sie, wenn ein Mobiltelefon an die Vorrichtung angesteckt wird, durch eine Batterie des Mobiltelefons gespeist wird und das Drahtlosmodul aktiviert, das mit dem Mobiltelefon gekoppelt ist, um die Leuchtdioden zu steuern.

2. Vorrichtung nach Anspruch 1, wobei die Vorrichtung ferner eine Klebstoffträgerschicht auf dem flexiblen Substrat umfasst, um einen Aufkleber zu bilden.

3. Vorrichtung nach Anspruch 1, ferner umfassend eine flexible Batterie, die mit der einen oder den mehreren Schichten gekoppelt ist.

4. Verfahren, umfassend:
Bereitstellen (402) eines flexiblen Substrats (110), das eine Basis für ein oder mehrere elektronische Bauelemente (112, 122, 132) umfasst; und
Bereitstellen (404) einer oder mehrerer Schichten (120, 130) auf dem flexiblen Substrat (110), wobei die eine oder die mehreren Schichten (120, 130) Komponenten für das eine oder die mehreren elektronischen Bauelemente (112, 122, 132) bilden,
wobei eine obere Schicht (130) der einen oder der mehreren Schichten (120, 130) zu einer Umgebung um die obere Schicht (130) freiliegend ist und Komponenten zum Interagieren mit der Umgebung umfasst, wobei eine zweite Schicht der einen oder der mehreren Schichten eine Induktionsleistungsspule umfasst, die zum Empfangen von Leistung zum Betreiben des einen oder der mehreren elektronischen Bauelemente ausgelegt ist, und wobei die obere Schicht der einen oder der mehreren Schichten eine oder mehrere Leuchtdioden umfasst, wobei die Leuchtdioden zum Empfangen von Leistung von der Induktionsleistungsspule ausgelegt sind, wobei eine erste Schicht der einen oder der mehreren Schichten ein Drahtlosmodul umfasst, und wobei das Drahtlosmodul so ausgelegt ist, dass es Befehle zum Betreiben der Leuchtdioden empfängt und die Leuchtdioden wenigstens zum Teil basierend auf den empfangenen Befehlen betreibt,
wobei die Leistungsspule so konfiguriert ist, dass sie, wenn ein Mobiltelefon an die Vorrichtung angesteckt wird, durch eine Batterie des Mobiltelefons gespeist wird und das Drahtlosmodul aktiviert, das mit dem Mobiltelefon gekoppelt ist, um die Leuchtdioden zu steuern.

5. Verfahren Anspruch 4, ferner umfassend ein Bereitstellen einer Klebstoffträgerschicht auf dem flexiblen Substrat, um einen Aufkleber zu bilden.

6. Verfahren nach Anspruch 4, ferner umfassend ein Koppeln einer flexiblen Batterie mit der einen oder den mehreren Schichten.

7. Verfahren nach Anspruch 4, ferner umfassend ein Verkappen des flexiblen Substrats unter Verwendung von Polymer-auf-Cellulose-Herstellung mittels 3D-Druck.

## Revendications

1. Appareil (100), comprenant :
un substrat flexible (110) comprenant une base pour un ou plusieurs dispositifs électroniques (112, 122, 132) ; et
une ou plusieurs couches (120, 130) sur le substrat flexible (110), dans lequel les une ou plusieurs couches forment des composants pour les un ou plusieurs dispositifs électroniques (112, 122, 132),
dans lequel une couche supérieure (130) des une ou plusieurs couches (120, 130) est exposée à un environnement autour de l'appareil (100) et inclut des composants pour interagir avec l'environnement, dans lequel une seconde couche des une ou plusieurs couches comprend une bobine d'alimentation inductive conçue pour recevoir de la puissance pour faire fonctionner les un ou plusieurs dispositifs électroniques, dans lequel la couche supérieure des une ou plusieurs couches comprend une ou plusieurs diodes électroluminescentes, dans lequel les diodes électroluminescentes sont conçues pour recevoir de la puissance de la bobine d'alimentation inductive, dans lequel une première couche des une ou plusieurs couches comprend un module sans fil, dans lequel le module sans fil est couplé à la bobine d'alimentation inductive et aux diodes électroluminescentes, dans lequel le module sans fil est conçu pour recevoir des ordres pour faire fonctionner les diodes électroluminescentes et pour faire fonctionner les diodes électroluminescentes sur la base, au moins en partie, des ordres reçus, et dans lequel, lorsqu'un téléphone mobile est branché sur l'appareil, la bobine d'alimentation est conçue pour être excitée par une batterie du téléphone mobile et pour activer le module sans fil, qui est associé au téléphone mobile pour commander les diodes électroluminescentes.

2. Appareil selon la revendication 1, dans lequel l'appareil comprend en outre un support adhésif sur le substrat flexible pour former un autocollant.

3. Appareil selon la revendication 1, comprenant en outre une batterie flexible couplée aux une ou plusieurs couches.

4. Procédé comprenant :
la fourniture (402) d'un substrat flexible (110) comprenant une base pour un ou plusieurs dispositifs électroniques (112, 122, 132) ; et
la fourniture (404) d'une ou plusieurs couches (120, 130) sur le substrat flexible (110), dans lequel les une ou plusieurs couches (120, 130) forment des composants pour les un ou plusieurs dispositifs électroniques (112, 122, 132),
dans lequel une couche supérieure (130) des une ou plusieurs couches (120, 130) est exposée à un environnement autour de la couche supérieure (130) et inclut des composants pour interagir avec l'environnement, dans lequel une seconde couche des une ou plusieurs couches comprend une bobine d'alimentation inductive conçue pour recevoir de la puissance pour faire fonctionner les un ou plusieurs dispositifs électroniques, et dans lequel la couche supérieure des une ou plusieurs couches comprend une ou plusieurs diodes électroluminescentes, dans lequel les diodes électroluminescentes sont conçues pour recevoir de la puissance de la bobine d'alimentation inductive, dans lequel une première couche des une ou plusieurs couches comprend un module sans fil, dans lequel le module sans fil est couplé à la bobine d'alimentation inductive et aux diodes électroluminescentes, et dans lequel le module sans fil est conçu pour recevoir des ordres pour faire fonctionner les diodes électroluminescentes et pour faire fonctionner les diodes électroluminescentes sur la base, au moins en partie, des ordres reçus,
dans lequel, lorsqu'un téléphone mobile est branché sur l'appareil, la bobine d'alimentation est conçue pour être excitée par une batterie du téléphone mobile et pour activer le module sans fil, qui est associé au téléphone mobile pour commander les diodes électroluminescentes.

5. Procédé selon la revendication 4, comprenant en outre la fourniture d'un support adhésif sur le substrat flexible pour former un autocollant.

6. Procédé selon la revendication 4, comprenant en outre le couplage d'une batterie flexible aux une ou plusieurs couches.

7. Procédé selon la revendication 4, comprenant en outre le conditionnement du substrat flexible au moyen d'une fabrication de polymère sur cellulose au moyen d'une impression 3D.
